# EUROPÄISCHE PATENTSCHRIFT

(11) **EP 0 916 365 B1**
(45) Veröffentlichungstag und Bekanntmachung des Hinweises auf die Patenterteilung: **28.07.2004**
(21) Anmeldenummer: 98120757.4
(22) Anmeldetag: 02.11.1998
(51) Int. Cl.: A61N 1/39

(54) **Defibrillator mit verbesserter Ausnutzung der Akkumulatorenergie**
Defibrillator with improved use of the battery energy
Défibrillateur à utilisation améliorée de l'énergie de l'accumulateur

(30) Priorität: 14.11.1997 DE 19750634
(43) Veröffentlichungstag der Anmeldung: 19.05.1999
(73) Patentinhaber: GE Medical Systems Information Technologies GmbH, 79111 Freiburg i. Br. (DE)
(72) Erfinder: Magin, Thomas, 79224 Umkirch (DE)
(74) Vertreter: Müller - Hoffmann & Partner

(56) Entgegenhaltungen:
- EP-A- 0 598 225
- WO-A-95/11058
- DE-A- 3 111 116
- GB-A- 2 265 312
- US-A- 4 548 209
- US-A- 5 115 807
- US-A- 5 265 588
- US-A- 5 488 553

## Beschreibung

Die vorliegende Erfindung betrifft einen Defibrillator mit einem über einen Gleichstrom-Gleichstrom-Wandler von einem Akkumulator aufladbaren Kondensator.

Ein solcher Defibrillator ist beispielsweise aus US 5 609 618 A bekannt.

Defibrillatoren können bekanntlich bei Herzrhythmusstörungen elektrische Stromstöße mit einer Energie von 2 bis 500 J und einer Zeitdauer von ungefähr 4 bis 8ms an den Körper eines Patienten abgeben. Externe oder transthorakale Defibrillatoren sind dabei tragbare Geräte, die ihre Energie von einem Akkumulator beziehen. Infolge ihrer Tragbarkeit dürfen diese Defibrillatoren kein zu großes Gewicht aufweisen, was wiederum bedeutet, daß die in die Defibrillatoren eingebauten Akkumulatoren nur über eine begrenzte Kapazität verfügen.

Der an den Körper des Patienten abzugebende Stromstoß wird durch die Entladung eines zuvor von dem Akkumulator unter Zwischenschaltung eines Gleichstrom-Gleichstrom-(DC-DC) Wandlers aufgeladenen Kondensators erzeugt. Bei Nachlassen der Energie des Akkumulators sinkt nun dessen Ausgangsspannung während der Aufladung des Kondensators schließlich so weit ab, daß die Restenergie im Akkumulator nicht mehr ausgenutzt werden kann, da sich der Defibrillator zuvor abschaltet.

Darüberhinaus wird die bereits im Kondensator des Defibrillators gespeicherte elektrische Energie intern im Defibrillator ohne Abgabe eines Stromimpulses an den Patienten vernichtet, weil dies durch Sicherheitsvorschriften so festgelegt ist: Im Kondensator darf nach Ablauf einer bestimmten Zeit keine Restenergie zurückbleiben, weil dies eine Gefahr darstellt.

Auch sind derzeit Defibrillatoren so aufgebaut, daß ein Bediener, also ein Arzt oder Sanitäter, die gewünschte Energie am Defibrillator einstellt. Ist der Kondensator des Defibrillators bis zu dieser Energie aufgeladen, so meldet der Defibrillator dies durch ein optisches/akustisches Signal und der Bediener kann den Stromstoß durch den Körper des Patienten freigeben. Kann diese eingestellte Energie aber infolge einer abnehmenden Akkumulatorenergie nicht erreicht werden, so wird nach einiger Zeit infolge der erwähnten Sicherheitsvorschriften die im Kondensator des Defibrillators gespeicherte Energie intern vernichtet.

In der Praxis ist es nun äußerst unbefriedigend, wenn der Kondensator eines Defibrillators infolge einer zu Ende gehenden Akkumulatorenergie nicht mehr bis zu der gewünschten Energie aufgeladen werden kann, da die noch im Akkumulator enthaltene Restenergie nicht auszunutzen ist. Gleiches gilt für einen nicht vollständig auf die gewünschte Energie aufgeladenen Kondensator. Es ist in vielen Fällen sicher besser, beispielsweise nur 90 % der gewünschten Energie aus einem Defibrillator an einen Patienten abzugeben, als vollständig auf eine Defibrillation zu verzichten oder diese erst nach einem längeren Zeitraum nach Ausrüsten des Defibrillators mit einem voll geladenen Akkumulator zur Anwendung zu bringen.

Zur Aufrechterhaltung eines sicheren Betriebszustandes bei einem implantierbaren Stimulationsgerät, insbesondere Herzschrittmacher, mit zunehmender Erschöpfung der Energiequelle ist es aus US 5 031 619 bekannt, bei sinkender Batteriespannung, d. h. steigendem Innenwiderstand, einen geringeren Strom zu entnehmen. Dabei wird der entnommene Strom so bemessen, daß die Spannung gerade oberhalb eines vorgegebenen Schwellwertes liegt und damit annähernd konstant gehalten ist.

Aus der US-A-5 488 553 ist ein Defibrillator gemäß dem Oberbegriff des Patentanspruchs bekannt; dieser besitzt einen über einen DC-DC-Wandler von einer Batterie aufladbaren Kondensator, wobei eine Steuereinrichtung vorgesehen ist, die mit sinkender Batteriespannung einen geringeren Strom entnimmt, so dass der Kondensator langsamer aufgeladen wird. Allerdings handelt es sich dort um einen implantierbaren, mit einem Herzschrittmacher kombinierten Defibrillator, bei dem das Problem einer Zwangsabschaltung bei ungenügender Aufladung des Kondensators keine Rolle spielt.

Aus der US-A-5 115 807 ist ein zweistufiger Defibrillator mit zwei getrennt einstellbaren Kondensatoren zur Anwendung bei der Untersuchung von Patienten in einem elektrophysikalischen Labor bekannt. Dort ist unter anderem eine Anzeigeeinrichtung vorgesehen, um in einer Testreihe unter anderem die gelieferte Energiemenge nach einem Defibrillationsschock, die Restenergie in dem Kondensator nach dem Schock, aber auch die bei einem Schock abzugebende Energie in Abhängigkeit von dem geschätzten elektrischen Widerstand des Patienten anzuzeigen. Das Problem eines unzureichend aufgeladenen Kondensators ist dort nicht angesprochen; außerdem wäre die dortige Apparatur für den Einsatz in einem tragbaren Notfall-Defibrillator nicht geeignet.

Es ist Aufgabe der vorliegenden Erfindung, einen Defibrillator zu schaffen, der in optimaler Weise die Restenergie eines Akkumulators auszunutzen vermag.

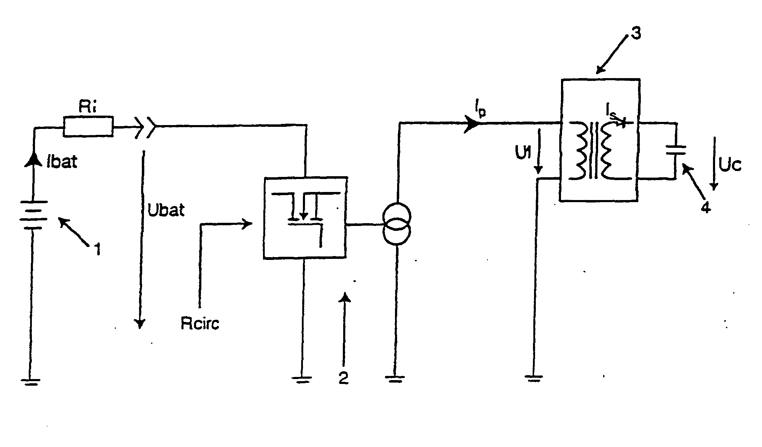

Diese Aufgabe wird erfindungsgemäß bei einem Defibrillator der eingangs genannten Art mit einem von einem Akkumulator oder einer Batterie über einen DC-DC-Wandler auf eine vorgegebene gewünschte Energiemenge aufladbaren Kondensator und mit einer zwischen dem Akkumulator und dem Kondensator vorhandenen Steuereinrichtung, welche die Ausgangsspannung des Akkumulators überwacht und bei einem Absinken der Ausgangsspannung des Akkumulators unter ihren nominellen Wert die Stromentnahme reduziert, dadurch gelöst, dass der Defibrillator ein tragbarer externer Defibrillator ist, der bei einem Absinken der Ausgangsspannung unter einen kritischen Wert automatisch abgeschaltet wird und bei dem dann die in dem Kondensator gespeicherte Restenergie intern vernichtet wird, und dass dann, wenn die Ausgangsspannung in Richtung auf den kritischen Wert sinkt, ohne dass der Kondensator auf die gewünschte Energiemenge aufgeladen werden kann, vor dem Abschalten des Defibrillators durch die Steuereinrichtung die in dem Kondensator gespeicherte Energie angezeigt und für eine Nutzung zur Verfügung gestellt wird. Nachfolgend wird die Erfindung anhand der Zeichnung näher erläutert, in deren einziger Figur ein schematisches Schaltbild des erfindungsgemäßen Defibrillators gezeigt ist.

Die Figur zeigt einen Akkumulator 1 mit einem Innenwiderstand Ri, eine Steuereinrichtung aus einer gesteuerten Stromquelle 2, einen DC-DC-Wandler 3 und einen Kondensator 4.

Bei einer Konstantstromladung des Kondensators 4 sind die folgenden Zusammenhänge zu beachten:

Wenn der Sekundärstrom Is des DC-DC-Wandlers 3 für die Aufladung des Kondensators konstant gehalten wird, steigt die Spannung Uc am Kondensator 4 proportional mit der abgelaufenen Ladezeit t an. Da sich die durch den DC-DC-Wandler 3 zu übertragende Leistung P aus dem Produkt der Kondensatorspannung Uc mit dem Sekundärstrom Is ergibt, steigt diese Leistung P ebenfalls proportional mit der abgelaufenen Ladezeit t an.

Der Primärstrom Ip des im DC-DC-Wandler 3 enthaltenen Transformators ist bekenntlich direkt proportional zum Sekundärstrom Is. Entsprechend sind beide Ströme Ip und Is konstant. Daher muß die ansteuernde Spannung U1 an der Primärseite des Transformators ebenfalls proportional mit der abgelaufenen Ladezeit t ansteigen.

Die der Konstantstromquelle 2 entnommene Leistung entspricht in erster Näherung der übertragenen Leistung P, sie ist also proportional zur abgelaufenen Ladezeit t.

Für die Spannungsversorgung aus dem Akkumulator 1 kann in erster Näherung von einer Konstantspannungsquelle ausgegangen werden, deren Innenwiderstand Ri klein gegenüber dem Schaltungswiderstand Rcirc ist. Wird nun dem Akkumulator 1 eine mit der Ladedauer ansteigende Leistung entnommen, so führt dies zu einem ebenfalls ansteigenden Akkumulatorstrom I_{bat}, da, wie vorrausgesetzt wurde, die Klemmenspannung U_{bat} des Akkumulators 1 konstant bleibt.

Damit ergibt sich, daß der Akkumulatorstrom I_{bat} in erster Näherung proportional zu der abgelaufenen Ladezeit t des Kondensators 4 ist.

Sinkt nun die im Akkumulator 1 gespeicherte Energie, so kann nicht mehr von einem konstanten Innenwiderstand Ri ausgegangen werden. Vielmehr steigt der Innenwiderstand Ri des Akkumulators 1 mit dessen sinkender Energie an. Zusammen mit dem ansteigenden Akkumulatorstrom I_{bat} führt dies zu einem Spannungsabfall über dem Innenwiderstand Ri während der Ladephase, der nicht zu vernachlässigen ist.

Sinkt aber bei bisherigen Defibrillatoren die Akkumulatorspannung U_{bat} unter einen kritischen Wert, so schaltet der Defibrillator automatisch ab, und die bereits im Kondensator 4 gespeicherte Energie wird intern vernichtet.

Ein Einsatz des Defibrillators ist erst dann möglich, wenn der Akkumulator 1 durch einen anderen Akkumulator ersetzt wird, der ausreichend aufgeladen ist. Hierfür ist wenigstens eine Zeitdauer von 20 Sekunden erforderlich, was oft für einen Patienten äußerst kritisch sein kann.

Das automatische Abschalten des Defibrillators kann verhindert werden, wenn bei steigendem Innenwiderstand Ri des Akkumulators 1 diesem ein geringerer Strom entnommen wird. Damit kann dann der Spannungsabfall über dem Innenwiderstand Ri konstant gehalten werden, was wiederum zu einer konstanten Akkumulator- bzw. Klemmenspannung U_{bat} führt.

Bei dem erfindungsgemäßen Defibrillator wird ausgenutzt, daß sich ein steigender Innenwiderstand Ri des Akkumulators 1 durch eine sinkende Akkumulatorspannung U_{bat} bemerkbar macht: Es wird also die Akkumulatorspannung U_{bat} überwacht. Ausgehend von einem nominellen Wert U_{bat}, für den dem Akkumulator 1 der maximale Strom entnommen werden kann, wird der Akkumulatorstrom I_{bat} automatisch so nachgeführt, daß die Akkumulatorspannung U_{bat} nur so weit absinkt, daß ein weiterer Betrieb des Defibrillators, gewährleistet ist.

Bei dem erfindungsgemäßen Defibrillator wird also die Klemmenspannung U_{bat} des Akkumulators überwacht. Ausgehend von dem erfaßten Wert für die Klemmenspannung wird der Akkumulatorstrom so nachgeführt, daß die Klemmenspannung U_{bat} nur so weit absinkt, daß noch ein Laden des Kondensators 4 möglich ist.

Dadurch wird zwar die Ladezeit t des Kondensators 4 verlängert. Es ist aber möglich, einem fast entladenen Akkumulator Restenergie zu entnehmen.

Ein derartiges Vorgehen ist natürlich nur in den Grenzen der im Akkumulator 1 verbliebenen Restenergie möglich. Sollte daher die im Akkumulator 1 verbliebene Restenergie nicht mehr ausreichend sein, um den Kondensator 4 vollständig auf die gewählte Energie zu laden, so wird der Ladevorgang abgebrochen bevor der Defibrillator automatisch abschaltet. Der Kondensator 4 weist also dann nicht die von dem Bediener gewünschte Energiemenge, sondern einen darunterliegenden Wert auf. Dieser darunterliegende Wert wird dem Benutzer angezeigt und zur Verfügung gestellt.

Die Restenergiemenge kann so an einen Patienten abgegeben werden, was insbesondere dann vorteilhaft ist, wenn der Kondensator 4 beispielsweise auf 90 % der gewünschten Energie aufgeladen ist, bevor der Defibrillator automatisch abschaltet. Diese Energiemengen von 90 % der gewünschten Energie können nämlich therapeutisch sehr wirksam sein und stehen insbesondere viel rascher zur Verfügung, als wenn zuvor ein Akkumulatorwechsel oder eine Neuladung des Akkumulators vorgenommen wird.

Anstelle eines Akkumulators kann selbstverständlich in gleicher Weise auch eine Batterie verwendet werden.

## Patentansprüche

1. Defibrillator mit einem von einem Akkumulator (1) oder einer Batterie über einen DC-DC-Wandler (3) auf eine vorgegebene gewünschte Energiemenge aufladbaren Kondensator (4) und mit einer zwischen dem Akkumulator (1) und dem Kondensator (4) vorhandenen Steuereinrichtung (2), welche die Ausgangsspannung (U_{bat}) des Akkumulators (1) überwacht und bei einem Absinken der Ausgangsspannung des Akkumulators (1) unter ihren nominellen Wert (U_{bat}) die Stromentnahme (I_{bat}) reduziert,
**dadurch gekennzeichnet,**
**dass** der Defibrillator ein tragbarer externer Defibrillator ist, der bei einem Absinken der Ausgangsspannung (U_{bat}) unter einen kritischen Wert automatisch abgeschaltet wird und bei dem dann die in dem Kondensator gespeicherte Restenergie intern vernichtet wird, und
**dass** dann, wenn die Ausgangsspannung in Richtung auf den kritischen Wert sinkt, ohne dass der Kondensator (4) auf die gewünschte Energiemenge aufgeladen werden kann, vor dem Abschalten des Defibrillators durch die Steuereinrichtung (2) die in dem Kondensator (4) gespeicherte Energie angezeigt und für eine Nutzung zur Verfügung gestellt wird.

## Claims

1. A defibrillator having a capacitor (4) which is chargeable via a DC-DC converter (3) by an accumulator (1) or a battery to a predetermined desired quantity of energy, and a control device (2) situated between the accumulator (1) and the capacitor (4), which control device (2) monitors the output voltage (U_{bat}) of the accumulator (1) and, as the output voltage of the accumulator (1) decreases below its nominal value (U_{bat}), reduces the current drain (I_{bat}),
**characterized in**
**that** the defibrillator is a portable external defibrillator which is automatically deactivated as the output voltage (U_{bat}) decreases below a critical value, the residual energy stored in the capacitor being destroyed internally in this case, and
**that** in the case the output voltage decreases toward its critical value without the capacitor (4) being chargeable to the desired quantity of energy, before deactivation of the defibrillator by the control device (2), the energy stored in the capacitor (4) is indicated and made available for utilization thereof.

## Revendications

1. Défibrillateur avec un condensateur (4) pouvant être chargé à une quantité d'énergie désirée prédéterminée par un accumulateur (1) ou une batterie par l'intermédiaire d'un convertisseur à courant continu (3) et avec un dispositif de commande (2) monté entre l'accumulateur (1) et le condensateur (4), qui surveille la tension initiale (U_{bat}) de l'accumulateur (1) et qui réduit l'intensité du courant prélevé (I_{bat}) lors d'une baisse de la tension initiale de l'accumulateur (1) en dessous de sa valeur nominale (U_{bat}), **caractérisé en ce que** le défibrillateur est un défibrillateur externe portable, qui est automatiquement déconnecté lors d'une baisse de la tension initiale (U_{bat}) en dessous d'une valeur critique et dans lequel l'énergie résiduelle accumulée dans le condensateur est alors consommée de manière interne, et **en ce que**, lorsque la tension initiale baisse en direction de la valeur critique sans que le condensateur (4) puisse être chargé à la quantité d'énergie désirée, l'énergie accumulée dans le condensateur (4) est affichée et est rendue disponible pour une utilisation avant que le défibrillateur soit déconnecté par le dispositif de commande (2).
